# EUROPÄISCHE PATENTANMELDUNG

(11) **EP 0 807 411 A1**
(43) Veröffentlichungstag der Anmeldung: **19.11.1997**
(21) Anmeldenummer: 97103314.7
(22) Anmeldetag: 28.02.1997
(51) Int. Cl.: A61B 17/32

(54) **Endoskopisches Instrument**

(30) Priorität: 17.05.1996 DE 19619970
(71) Anmelder: Richard Wolf GmbH, 75438 Knittlingen (DE)
(72) Erfinder: Heimberger, Rudolf, 75038 Oberderdingen (DE)
(74) Vertreter: Vollmann, Heiko, Dipl.-Ing.

(57) **Zusammenfassung**

Das endoskopische Instrument (1) weist einen Schaft (2) und eine durch diesen Schaft (2) verlaufende antreibbare Hohlwelle (3) auf. Durch die Hohlwelle (3) sind Partikel und/oder Flüssigkeit vom distalen zum proximalen Instrumententeil führbar oder umgekehrt. Das Instrument (1) weist ein am distalen Ende angeordnetes wechselbares Werkzeug (5) auf. Um das Werkzeug (5) austauschen zu können, ist es lösbar jedoch formschlüssig drehfest mit dem Wellenende, bzw. mit dem Schaft (bei einem mehrteiligen Werkzeug) verbunden.

## Beschreibung

Die Erfindung betrifft ein endoskopisches Instrument zur medizinischen und/oder technischen Anwendung mit einem Schaft und einer durch diesen Schaft verlaufenden antreibbaren Hohlwelle, durch die Partikel und/oder Flüssigkeit vom distalen zum proximalen Instrumententeil führbar sind oder umgekehrt, sowie mit einem am distalen Instrumentenende angeordneten Werkzeug.

Eine ganze Reihe von derartigen Instrumenten für problematisch zu erreichende Körperregionen sind schon bekannt. Sie werden für verschiedene therapeutische Maßnahmen wie zum Beispiel das Abtragen von Knochen oder Knorpel verwendet. Das am distalen Ende des Instrumentes angeordnete Werkzeug kann ein Messer, Fräser, Schleifer oder dergleichen sein.

Eine Mehrzahl von therapeutischen Maßnahmen benötigen auch eine entsprechende Anzahl von solchen Instrumenten mit einem dem Zweck entsprechenden, am distalen Instrumentenende angeordneten Werkzeug. Bei den bekannten Instrumenten ist das Werkzeug mit dem Instrumentenende fest verbunden. Sind unterschiedliche Werkzeuge für verschiedene therapeutische Maßnahmen erforderlich, zum Beispiel Fräser, Schneider usw., dann muß der mit dem Motorhandgriff gekuppelte Instrumententeil komplett ausgetauscht werden. Damit die Anwender die erforderlichen therapeutischen Maßnahmen ausführen können, führt dies in vielen Fällen dazu, daß zahlreiche derartige Instrumente bzw. Instrumententeile benötigt werden, was aufgrund erforderlicher Bevorratung eines umfangreiches Instrumentariums dementsprechend erheblichen Kostenaufwand bedingt.

Im übrigen ist die Wartung oder das Nachbearbeiten der Werkzeuge sehr kostenaufwendig, da diese Werkzeugteile aufeinander abgestimmt sein müssen, um beispielsweise einen sauberen und effektiven Schnitt zu erzielen. Ist das Werkzeug oder auch nur ein Werkzeugteil beschädigt, so muß in jedem Fall der zugehörige gesamte Instrumententeil ersetzt werden.

Hiervon ausgehend liegt der Erfindung die Aufgabe zugrunde, ein gattungsgemäßes Instrument so auszubilden, daß im Fall einer Beschädigung des Werkzeuges oder eines Werkzeugteiles die Möglichkeit des Austausches nur dieses Werkzeuges bzw. Werkzeugteiles besteht und dieser Austausch vom Anwender selbst vorgenommen werden kann. Darüberhinaus soll der Anwender die Möglichkeit erhalten, unterschiedliche Werkzeuge an ein und demselben Instrument anbringen zu können, um mit demselben Instrument unteschiedliche Aufgaben erfüllen zu können, wie dies bei der Durchführung eines endoskopischen Eingriffes regelmäßig erforderlich ist.

Diese Aufgabe wird erfindungsgemäß dadurch gelöst, daß mindestens ein Teil des Werkzeuges mit einem Teil des distalen Endes des Instrumentes lösbar, jedoch formschlüssig drehfest verbunden ist. Bei einteiligem Werkzeug ist also vorgesehen, dieses lösbar mit dem Wellenende zu verbinden, bei einem mehrteiligen Werkzeug entsprechend so, daß der eine Teil des Werkzeuges lösbar mit der Welle und der andere lösbar mit dem Schaft verbunden ist.

Durch diese erfindungsgemäße Ausbildung wird erreicht, daß das am distalen Ende des Instrumentes befindliche Werkzeug bei Bedarf sofort und ohne zusätzliche Hilfsmittel leicht vom Anwender selbst getauscht bzw. gewechselt werden kann. Darüberhinaus wird ermöglicht, mit nur einem Grundinstrument eine Vielzahl von therapeutischen Maßnahmen durch entsprechenden Werkzeugwechsel ausführen zu können. Zusätzlich werden die Instrumentenkosten gering gehalten.

Zweckmäßigerweise sind das Werkzeug oder die Werkzeugteile in einer rohrförmigen Werkzeugaufnahme angeordnet, deren proximales Ende mit dem Schaftende formschlüssig verbunden ist.

Für Werkzeuge, die aus zwei relativ zueinander bewegbaren Werkzeugteilen bestehen wie zum Beispiel Messer mit Gegenschneide, ist es vorteilhaft, wenn ein Werkzeugteil zwischen dem Werkzeug und der Werkzeugaufnahme feststehend angeordnet ist, wobei der feststehende Werkzeugteil formschlüssig lösbar mit der Werkzeugaufnahme verbunden ist.

Vorzugsweise ist diese formschlüssig lösbare Verbindung zwischen dem feststehenden Werkzeugteil und der Werkzeugaufnahme durch mindestens einen am feststehenden Werkzeugteil proximalseitig außen angeordneten Querstift und eine entsprechend an der Innenseite der Werkzeugaufnahme vorgesehene Nut für diesen Querstift gebildet. Eine entsprechende Verbindung kann auch zwischen dem rotierbaren, also mit der Hohlwelle zu verbindenen Werkzeugteil und der Hohlwelle erfolgen. Wenn das Werkzeug bzw. der rotierbare Werkzeugteil, wie dies in der Regel üblich ist, nur für den Antrieb in eine Drehrichtung vorgesehen ist, wird die Formschlußverbindung zwischen Welle und Werkzeug bzw. zwischen Welle und Werkzeugteil bevorzugt als Gewindeverbindung ausgebildet, da hierdurch eine sichere Verbindung bei schlanker Bauweise und großem Innenlumen erhalten wird.

Damit die distale Instrumentenspitze so ausgebildet ist, daß sowohl eine Funktion als Gleitlager als auch die Aufnahme von feststehenden Komponenten möglich ist, bildet die Werkzeugaufnahme vorzugsweise Teil eines Radiallagers.

Damit kleine sowie größere Werkzeuge, beispielsweise Fräser unterschiedlicher Größe an demselben Instrument eingesetzt werden können, ist es erforderlich, daß diese einen proximalseitigen zylindrisch ausgebildeten Endteil mit einer nach außen gerichteten Ringschulter aufweisen, die sich dann am distalen oder proximalen stirnseiten Ende der am Außenschaft angeordneten Werkzeugaufnahme abstützen kann.

Ferner weist ein derartiger Fräser, um abgerennte Gewebeteile oder dergleichen abführen sowie um Spülflüssigkeit in die Körperhöhle zu- und abführen zu können, eine vorzugsweise seitlich angeordnete Ausnehmung auf, die in den zentralen Kanal der Welle mündet.

Nach dem Entfernen des Werkzeuges und der im Außenschaft drehbar gelagerten Hohlwelle kann der verbleibende Schaft, der auch als distalseitig ablenkbarer Schaft ausgebildet sein kann, zum Einführen sowie zum Steuern beliebiger Hilfsinstrumente verwendet werden. Dies ermöglicht dem Anwender beispielsweise vor oder nach Durchführung eines endoskopischen Eingriffes mittels einer flexiblen Optik zunächst entsprechende Untersuchungen vornehmen zu können.

Die Erfindung wird nachfolgend anhand von in den Zeichnungen dargestellten Ausführungsbeispielen näher erläutert. Es zeigen:
- Fig. 1: einen Längsschnitt durch das distale Ende eines erfindungsgemäßen Instrumentes mit einem als Messer ausgebildeten Werkzeug und einem als Gegenschneide dienenden zweiten Werkzeugteil,
- Fig. 2: einen Schnitt gemäß Linie II-II in Fig. 1,
- Fig. 3: eine Ausführungsvariante, bei der der zweite Werkzeugteil vom distalen Ende her in die Werkzeugaufnahme einführbar und in dieser festlegbar ist in Darstellung nach Fig. 1,
- Fig. 4: eine weitere Ausführungsvariante zu der Ausführung mit einem als Fräser ausgebildeten einteiligen Werkzeug in Darstellung nach Fig. 1,
- Fig. 5: die Ausführungsvariante nach Fig. 4, jedoch mit einem als großen Fräser ausgebildeten Werkzeug in Darstellung nach Fig. 1 und
- Fig. 6: die Einzelheit Z aus Fig. 3 in vergrößerter Darstellung.

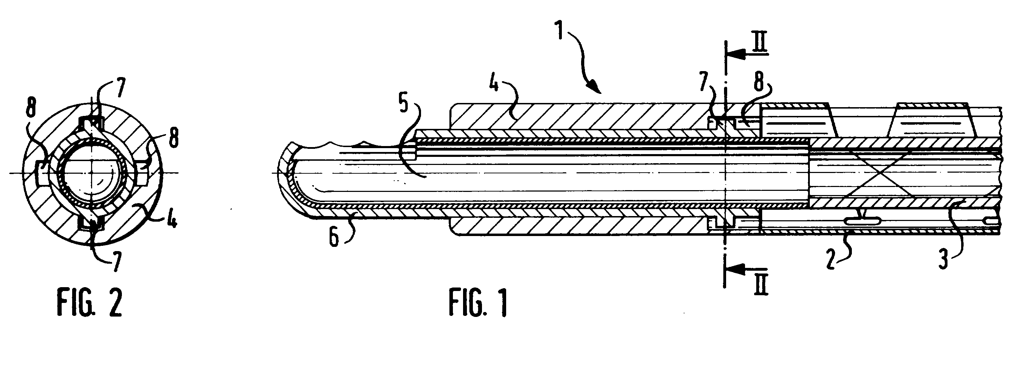

Fig. 1 zeigt das distale Ende des erfindungsgemäßen Instrumentes 1. Es besteht im wesentlichen aus einem hohlzylindrischen Schaft 2, einer durch den Schaft 2 verlaufenden antreibbaren Hohlwelle 3, einer am distalen Ende des Schaftes 2 angeordnete Werkzeugaufnahme 4 und einem ebenfalls am distalen Ende der Welle 3 angeordneten Werkzeug 5, das in dieser Ausführung ein Messer ist. Das Werkzeug 5 ist zweiteilig aufgebaut und besteht aus einem ersten rotierbaren Werkzeugteil, dem Messer und einem zweiten feststehenden Werkzeugteil, einer Gegenschneide 6. Die zusätzliche Gegenschneide 6 ist formschlüssig aber lösbar innerhalb der Werkzeugaufnahme 4 angeordnet. Das Messer ist mit seinem proximalen Ende lösbar, jedoch formschlüssig mit dem distalen Wellenende verbunden. Das proximalseitige Instrumentenende ist in an sich bekannter Weise ausgebildet, es wird hierzu auf DE-GM 79 00 066 und DE-GM 80 30 149 verwiesen.

Der Außenschaft 2 und die Welle 3 sind hier flexibel und distal steuerbar ausgebildet, können jedoch auch starr sein. Die Welle 3 ist innerhalb des Schaftes 2 drehbar gelagert und hohl ausgebildet, so daß Partikel, Spülflüssigkeit oder dergleichen vom distalen zum proximalen Instrumententeil abführbar sind oder umgekehrt Spülflüssigkeit zuführbar ist. Die Werkzeugaufnahme 4 ist im wesentlichen rohrförmig ausgebildet und mit ihrem proximalen Endteil mit dem distalen Ende des Schaftes 2 fest verbunden.

Bei der in Fig. 1 gezeigten Ausführung ist der feststehende und die Gegenschneide 6 bildende Werkzeugteil von proximalwärts durch den Schaft 2 zum distalen Ende geführt, wo er formschlüssig in die Werkzeugaufnahme 4 eingegliedert und in der gewünschten Raststellung fixiert ist. Hierfür sind zwei Stifte 7 nahe dem proximalen Ende an der Außenseite der Gegenschneide angeordnet. In der Innenseite der Werkzeugaufnahme sind entsprechende Nuten 8 zur Aufnahme dieser Stifte 7 vorgesehen (Fig. 2).

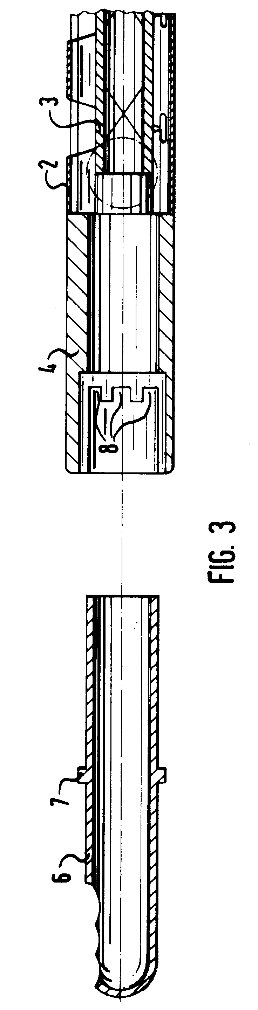

Bei der Ausführung nach Fig. 3 wird der zweite, die Gegenschneide 6 bildende Werkzeugteil vom distalen Instrumentenende her in die Werkzeugaufnahme 4 eingeführt, verdreht und dann distalwärts gezogen, um in eine von mehreren Raststellungen einzugreifen.

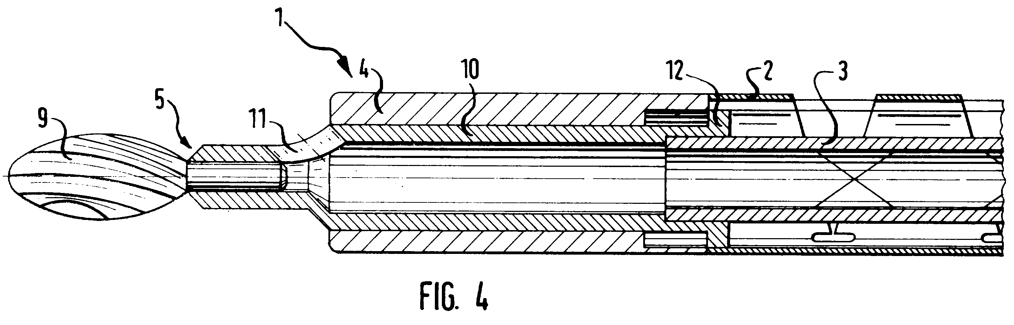

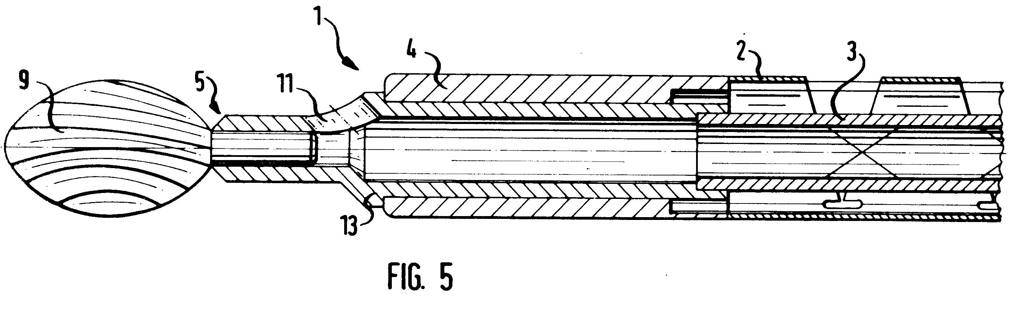

Die Figuren 4 und 5 zeigen eine weitere Ausführung des Instrumentes, das hier mit einem als Fräser 9 ausgebildeten einteiligen Werkzeug 5 ausgestattet ist. Der Fräskopf 9 zusammen mit einem damit fest verbundenen, im wesentlich rohrförmigen Schaft 10 bilden eine bauliche Einheit. Am distalen Ende des Schaftes 10 ist eine Ausnehmung 11 vorgesehen, die das Absaugen von Partikeln sowie das Zu- und Abführen von Spülflüssigkeit ermöglicht. Das Werkzeug 5 ist mit seinem Schaft 10 lösbar, jedoch formschlüssig drehfest mit dem distalen Ende der Hohlwelle 3 verbunden und innerhalb der als Radiallager ausgebildeten Werkzeugaufnahme 4 drehbar gelagert. Die Werkzeugaufnahme 4 ist wie bei der oben erwähnten Ausführung mit dem distalen Ende des Schaftes 2 fest verbunden. Am Schaft 10 ist bei der Ausführung nach Fig. 4 eine proximale bzw. nach Fig. 5 eine distale Außenschulter 12, 13 vorgesehen, die am distalen bzw. proximalen stirnseitigen Ende der Werkzeugaufnahme anliegt.

Bei dem in Fig. 4 gezeigten kleinen Fräskopf 9 wird das proximale Ende des Werkzeugs 5 formschlüssig drehfest mit der Welle 3 verbunden und distalwärts durch die Werkzeugaufnahme 4 geschoben, wobei die Schulter 12 des Lagers am proximalen Ende des Werkzeugs 5 anliegt.

Bei größeren Werkzeugen geschieht dies umgekehrt, wie aus Fig. 5 zu entnehmen ist. Hier erfolgt das Einführen des Werkzeuges 5 vom distalen Ende der Werkzeugaufnahme her, da das Werkzeug 5 größer ist als der Innendurchmesser der Werkzeugaufnahme 4. Hier liegt die Schulter 13 des Lagers am distalen Ende der Werkzeugaufnahme 4 an.

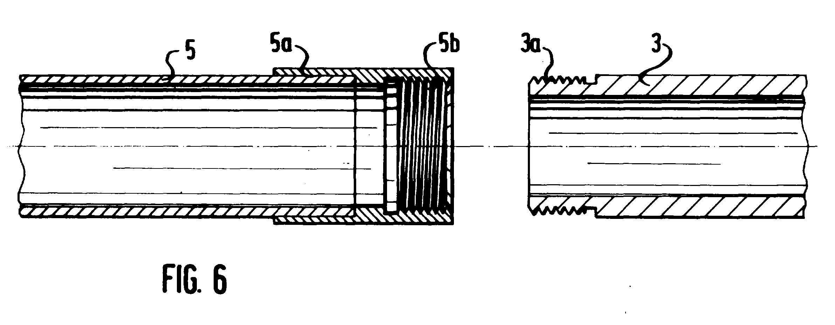

Bei allen Ausführungsvarianten ist das Werkzeug 5 bzw. der drehfest mit der Welle 3 verbundene Werkzeugteil lösbar mit der Hohlwelle 3 verbunden. Hierzu ist im distalen Bereich, etwa in Höhe der Aufnahme 4 eine Gewindeverbindung vorgesehen, wie sie in Fig. 6 im einzelnen dargestellt ist. Das distale Ende der Welle 3 ist hierzu mit einem Außengewinde 3a versehen, das in ein entsprechendes Innengewinde 5b am proximalen Ende 5a des Werkzeuges 5 eingreift. Da die Wandung des Werkzeuges 5 bzw. des Schaftes 10 vergleichsweise dünnwandig ausgebildet ist, ist das proximale Werkzeug- bzw. Schaftende durch eine Hülse 5a verstärkt. Die Hülse 5a kann durch Kleben oder vorzugsweise durch Löten mit dem Werkzeug 5 verbunden sein. Anstelle der Gewindeverbindung 3a, 5b kann auch eine Bajonettverbindung ähnlich der Verbindung zwischen der Werkzeugaufnahme 4 und dem zweiten Werkzeugteil 6 vorgesehen sein.

Wegen der Möglichkeit, das distalseitig angeordnete Werkzeug bei Bedarf wechseln zu können, ist die Verwendung nicht nur auf die medizinische Anwendung beschränkt, sondern kann auch bei entsprechender Dimensionierung der Abmessungen in der Technik beispielsweise zur Bearbeitung von Oberflächen technischer Hohlräume verwendet werden.

## Patentansprüche

1. Endoskopisches Instrument mit einem Schaft (2) und einer durch diesen Schaft (2) verlaufenden antreibbaren Hohlwelle (3), durch die Partikel und/oder Flüssigkeit vom distalen zum proximalen Instrumententeil führbar sind oder umgekehrt, sowie mit einem am distalen Instrumentenende angeordneten Werkzeug (5), dadurch gekennzeichnet, daß mindestens ein Teil des Werkzeuges (5, 6) mit einem Teil (2, 3) des distalen Endes des Instrumentes (1) lösbar, jedoch formschlüssig drehfest verbunden ist.

2. Endoskopisches Instrument nach Anspruch 1, dadurch gekennzeichnet, daß das Werkzeug (5) oder ein Werkzeugteil in einer rohrförmigen Werkzeugaufnahme (4) angeordnet ist, deren proximales Ende mit dem Schaftende formschlüssig verbunden ist.

3. Endoskopisches Instrument nach Anspruch 1 oder 2, dadurch gekennzeichnet, daß ein feststehendes zweites Werkzeugteil (6) zwischen dem ersten Werkzeugteil (5) und der Werkzeugaufnahme (4) angeordnet ist, wobei der zweite Werkzeugteil (6) formschlüssig lösbar mit der Werkzeugaufnahme (4) verbindbar ist.

4. Endoskopisches Instrument nach einem der vorhergehenden Ansprüche, dadurch gekennzeichnet, daß die formschlüssig lösbare Verbindung zwischen dem zweiten Werkzeugteil (6) und der Werkzeugaufnahme (4) über mindestens einen an der Außenseite und eine an der entsprechenden Innenseite dieser jeweiligen Teile angeordneten Stift (7) bzw. eine Nut (8) erfolgt.

5. Endoskopisches Instrument nach einem der vorhergehenden Ansprüche, dadurch gekennzeichnet, daß die Werkzeugaufnahme (4) zumindest Teil eines Radiallagers bildet.

6. Endoskopisches Instrument nach einem der vorhergehenden Ansprüche, dadurch gekennzeichnet daß das Werkzeug (5) im wesentlichen aus einem Werkzeugkopf (9) und einem rohrförmigen Teil (10) besteht, dessen Außendurchmesser dem Innendurchmesser der Werkzeugaufnahme (4) entspricht.

7. Endoskopisches Instrument nach Anspruch 6, dadurch gekennzeichnet, daß der rohrförmige Teil (10) mit einer proximalen oder distalen Schulter (12, 13) versehen ist, die an der Werkzeugaufnahme (4) angreift.

8. Endoskopisches Instrument nach einem der vorhergehenden Ansprüche, dadurch gekennzeichnet, daß eine Ausnehmung (11) nahe dem distalen Ende des Werkzeugs (5) vorgesehen ist, die das Durchfließen von Partikel und/oder Flüssigkeit ermöglicht.
